# FASCICULE DE BREVET EUROPEEN

(11) **EP 1 005 856 B1**
(45) Date de publication et mention de la délivrance du brevet: **13.02.2002**
(21) Numéro de dépôt: 99402742.3
(22) Date de dépôt: 04.11.1999
(51) Int. Cl.: A61K 7/48

(54) **COMPOSITION SOUS FORME D'EMULSION E/H A FORTE TENEUR EN CIRE ET SES UTILISATIONS DANS LES DOMAINES COSMETIQUE ET DERMATOLOGIQUE**
W/O-EMULSIONEN MIT HOHEM WACHSGEHALT UND DEREN VERWENDUNG IM KOSMETISCHEN UND DERMATOLOGISCHEN BEREICH
W/O-EMULSIONS WITH A HIGH WAX CONTENT AND THEIR USE IN THE COSMETIC AND DERMATOLOGIC FIELD

(30) Priorité: 03.12.1998 FR 9815292
(43) Date de publication de la demande: 07.06.2000
(73) Titulaire: L'OREAL, 75008 Paris (FR)
(72) Inventeur: Roulier, Véronique, 75010 Paris (FR); Daubige, Thérèse, 77480 Mousseaux les Bray (FR)
(74) Mandataire: Rasson, Catherine

(56) Documents cités:
- EP-A- 0 152 953
- US-A- 5 362 482

## Description

La présente invention se rapporte à une composition sous forme d'une crème constituée d'une émulsion eau-dans-huile (E/H) comportant une forte teneur en cire, et à ses utilisations dans les domaines cosmétique et dermatologique, notamment pour le soin, le traitement et/ou le maquillage de la peau et/ou des muqueuses, et plus particulièrement pour le traitement des rides et/ou ridules de la peau et/ou pour le traitement de la peau sèche.

L'invention concerne également un procédé de préparation de cette composition selon lequel on effectue au moins une étape du procédé à l'aide d'un mélangeur-extrudeur.

Il est connu d'utiliser des cires dans des crèmes cosmétiques se présentant sous forme d'émulsions et destinées au soin de la peau humaine, notamment pour les effets antirides apportés par ces cires. Néanmoins, il est difficile d'incorporer dans ces compositions un fort pourcentage de cires car les cires ont tendance à épaissir énormément les émulsions. En outre, quand on incorpore un fort pourcentage de cires dans une émulsion, celle-ci s'applique très difficilement sur la peau car elle ne glisse pas. De plus, il apparaît un effet rêche sur la peau. L'utilisation d'une telle émulsion est donc rédhibitoire pour l'utilisateur.

Par ailleurs, il est connu d'incorporer un fort pourcentage de cires dans des mascaras. Toutefois, ce type de compositions n'est pas utilisable comme produit de soin du fait des inconvénients précédemment cités.

Par ailleurs, pour préparer une émulsion contenant des cires, il est nécessaire de faire fondre les cires dans la phase grasse de l'émulsion, notamment si l'on veut par exemple utiliser des cires telles que les cires de carnauba qui sont particulièrement intéressantes pour leur effet antirides sur la peau. Il faut donc chauffer la phase grasse jusqu'à 80-85°C, ce qui est particulièrement préjudiciable lorsque l'on souhaite introduire des composés thermosensibles.

Il subsiste donc le besoin d'une composition ayant la consistance d'une crème et contenant un pourcentage important de cires sans les inconvénients de l'art antérieur.

La demanderesse a trouvé de manière surprenante que l'on pouvait incorporer un fort pourcentage de cires dans des crèmes sous forme d'émulsions E/H tout en conservant une fluidité satisfaisante et une sensation agréable lors de l'application sur la peau, en préparant l'émulsion à froid à l'aide d'un émulsionnant siliconé et à partir d'une phase huileuse souple contenant un fort pourcentage de cires.

Le document US-A-5,362,482 décrit des compositions solides sous forme d'émulsions eau-dans-huile contenant un tensioactif siliconé, et le document EP-A-152953 décrit des concentrés d'émulsifiants pour émulsions eau-dans-silicone volatile, contenant un tensioactif siliconé.

La présente invention a donc pour objet une composition sous forme d'une crème constituée d'une émulsion eau-dans-huile comportant une phase aqueuse dispersée dans une phase huileuse, caractérisée en ce qu'elle contient au moins 25 % en poids de phase aqueuse par rapport au poids total de la composition, au moins un émulsionnant siliconé et au moins 5% en poids d'une ou plusieurs cires par rapport an poids total de la composition, en ce que la phase huileuse se présente sous forme d'une pâte souple si la température ambiante, susceptible d'être obtenue en malaxant le mélange obtenu à partir du prémélange fondu des cires et huiles et des autres constituants de la phase huileuse tout en le refroidissant jusqu'a température ambiante et en ce que le mélange des phases huileuse et aqueuse se fait à froid.

La composition de l'invention est sous forme d'une crème, c'est-à-dire un produit souple par opposition à un produit solide tel qu'un stick. Une crème a une viscosité à la température ambiante (environ 20-25°C) allant d'environ 1 à 25 Pa.s et de préférence d'environ 1 à 10 Pa.s, cette viscosité étant mesurée avec un Rhéomat 180.

La composition de l'invention, bien que contenant un fort taux de cire, contient un taux important de phase aqueuse et est donc fraîche à l'application. En outre, le mélange de la phase aqueuse et de la phase huileuse étant fait à froid, on peut incorporer des composés thermosensibles sans craindre leur dégradation.

La composition de l'invention contient dans la phase huileuse, au moins 5 % en poids d'une ou plusieurs cires, par rapport au poids total de la composition. La phase huileuse refroidie avant son mélange avec la phase aqueuse se présenté de façon avantageuse sous forme d'une pâte souple à la température ambiante (environ 25°C). On entend ici par "pâte souple" une pâte dont on peut mesurer la viscosité, par opposition à la structure solide d'un bâton ou stick, dont on ne peut pas mesurer la viscosité. La viscosité dynamique de la pâte souple à 25°C est généralement comprise entre 3 et 35 Pa.s, mesurée avec un viscosimètre rotatif CONTRAVES TV équipé d'un mobile "MS-r4" à la fréquence de 60 Hz.

Comme cires utilisables dans la composition de l'invention, on peut citer par exemple les cires minérales telles que les cires microcristallines, la paraffine, le pétrolatum, la vaseline, l'ozokérite, la cire de montan ; les cires animales telles que la cire d'abeilles, la lanoline et ses dérivés ; les cires végétales telles que les cires de Candellila, d'Ouricurry, de Carnauba, du Japon, le beurre de cacao, les cires de fibres de lièges ou de canne à sucre ; les huiles hydrogénées concrètes à 25°C ; les esters gras et les glycérides concrets à 25°C ; les cires synthétiques telles que les cires de polyéthylène et les cires obtenues par synthèse de Fischer-Tropsch ; les cires de silicone, et leurs mélanges.

Selon un mode préféré de réalisation de l'invention, on utilise au moins une cire ayant une température de fusion commençante supérieure ou égale à 50°C, et mieux au moins une cire dont la température de fusion commençante est supérieure à 65°C, telles que la cire de Carnauba, certaines cires de polyéthylène et certaines cires microcristallines telles que celle vendue par la société Tisco sous le nom "Tisco Wax 88" ou celle vendue par la société RMC, sous le nom de "Feruwax 30540".

Par "température de fusion commençante", on entend dans la présente description la température à laquelle une cire commence à fondre. On peut déterminer cette température par ATD (analyse thermique différentielle) qui permet l'obtention du thermogramme (ou courbe de fusion) de la cire considérée. La température de fusion commençante correspond à la température à laquelle on peut observer un changement de pente notable dans le thermogramme. Le point de fusion, quant à lui, représente le point minimum dudit thermogramme.

La quantité de cire(s) dans la composition de l'invention est d'au moins 5 % et va de préférence de 5 à 30 % et mieux de 5 à 15 % en poids par rapport au poids total de la composition.

La quantité de phase huileuse contenant au moins une cire, dans la composition de l'invention va généralement de 20 à 75 % et de préférence de 30 à 60 % en poids par rapport au poids total de la composition. Cette phase huileuse est utilisée en une quantité telle ou bien contient une quantité de cires telle que la quantité de cires dans la composition finale est égale ou supérieure à 5 %.

La phase huileuse de la composition de l'invention comprend généralement, outre la ou les cires, un ou plusieurs corps gras choisis parmi les huiles d'origine animale, les huiles d'origine végétale, les huiles minérales, les huiles synthétiques, les huiles fluorées, les huiles de silicone et notamment les huiles de silicone volatiles, les gommes de silicone, les résines de silicone, les alcools gras, les acides gras et les élastomères de silicone tels que les produits commercialisés sous la dénomination "KSG" par la société Shin-Etsu, sous la dénomination "Trefil" par la société Dow Corning ou sous la dénomination "Gransil" par la société General Electric.

L'émulsionnant siliconé utilisé dans la composition de l'invention est généralement choisi dans le groupe comprenant les polydiméthylsiloxanes oxyéthylénés et/ou oxypropylénés, les alkyl-C₁₀-C₂₂-polydiméthylsiloxanes oxyéthylénés et/ou oxypropylénés ("alkyl-C₁₀-C₂₂" signifiant que la chaîne alkyle comporte de 10 à 22 atomes de carbone), les polydiméthylsiloxanes oxyéthylénés et/ou oxypropylénés à groupements glucosides, et leurs mélanges. Ces polydimethylsiloxanes peuvent être éventuellement réticulés.

L'émulsionnant siliconé peut être introduit tel quel ou en mélange avec une huile de silicone volatile ou non volatile, telle qu'une cyclométhicone (cyclohexasiloxane, cyclopentasiloxane, cyclotétrasiloxane).

On peut citer comme émulsionnant siliconé utilisable dans la composition de l'invention, les mélanges de dimethicone copolyol et de cyclomethicone vendus sous les dénominations "Q2-3225C" et "DC2-5225C" par la société Dow Corning, le produit vendu sous la dénomination "SF-1288" par la Société General Electric, le mélange de Polyglyceryl-4 isostearate/Cetyl dimethicone copolyol/Hexyl laurate vendu sous la dénomination "Abil WE 09" par la société Goldschmidt, le Cetyl dimethicone copolyol vendu sous la dénomination "Abil EM 90" par la société Goldschmidt, le Laurylmethicone copolyol vendu sous la dénomination "Q2-5200" par la société Dow Corning.

La quantité d'émulsionnant siliconé dans la composition selon l'invention va généralement de 0,1 à 10 % en poids en matière active et de préférence de 2 à 5 % en poids en matière active par rapport au poids total de la composition.

De manière avantageuse, la composition de l'invention peut aussi contenir une ou plusieurs charges (constituants pulvérulents) qui peuvent être choisies par exemple dans le groupe formé par le talc ; les micas d'origine naturelle ou synthétique ; le kaolin ; les oxydes de zinc ou de titane ; le carbonate de calcium ; le carbonate et l'hydrocarbonate de magnésium ; la silice, en particulier la silice sphérique, la poudre de silice commercialisée sous la dénomination "Cab-O-Sil TS 530" par la société Cabot, et les microbilles de silice telles que celles commercialisées sous la dénomination SB150 par la société Myoshi ; le dioxyde de titane ; les billes de verre et de céramique commercialisées par la société 3M sous la dénomination commerciale "Macrolite" ; les savons métalliques dérivés d'acide organique carboxylique ayant de 8 à 22 atomes de carbone, de préférence de 12 à 18 atomes de carbone, par exemple le stéarate de zinc, de magnésium ou de lithium, le laurate de zinc, le myristate de magnésium ; les poudres de polymères synthétiques non expansées, telles que les poudres de polyéthylène, de polystyrène, de polyesters, de polyamides (par exemple le nylon ou la poly-β-alanine), de copolymères d'acrylates (par exemple les microsphères microporeuses vendues par la société Dow Corning sous la dénomination commerciale "Polytrap"), d'acides polyméthacryliques, de polystyrène, de téflon comme le "Fluon" ; les poudres expansées telles que les microsphères creuses en matériau thermoplastique préparées par des procédés connus, comme ceux décrits dans US-A-3 615 972 et EP-A-0 56219 et notamment, les microsphères commercialisées sous la dénomination commerciale "Expancel" par la société Kemanord Plast ou sous la dénomination commerciale "Micropearl F 80 ED" par la société Matsumoto ; les poudres de matériaux organiques naturels tels que les amidons de maïs, de blé ou de riz, réticulés ou non, telles que les poudres d'amidon réticulé par l'anhydride octénylsuccinate, commercialisées sous la dénomination "Dry-Flo" par la société National Starch ; les microbilles de résine de silicone telles que celles commercialisées sous la dénomination "Tospearl" par la société Toshiba Silicone, et leurs mélanges.

Les charges peuvent représenter jusqu'à 20 % en poids par rapport au poids total de la composition, et de préférence de 1 à 12 % en poids par rapport au poids total de la composition.

La phase aqueuse de la composition de l'invention représente au moins 25 % en poids par rapport au poids total de la composition et de préférence de 40 à 70 % en poids par rapport au poids total de la composition.

La composition selon l'invention peut être utilisée dans. tous les domaines où ce type de forme galénique est intéressant, et notamment dans les domaines cosmétique et dermatologique. Quand elle constitue une composition cosmétique et/ou dermatologique, elle contient avantageusement un milieu physiologiquement acceptable, c'est-à-dire compatible avec la peau, les muqueuses, les ongles et/ou les cheveux.

Les compositions, objet de l'invention, trouvent leur application dans un grand nombre de traitements de la peau, des muqueuses (lèvres) et des cheveux, y compris le cuir chevelu, notamment pour la protection, le soin, le nettoyage et/ou le maquillage de la peau et/ou des muqueuses, pour la protection, le soin et/ou le nettoyage des cheveux et/ou pour le traitement de la peau, des cheveux et/ou des muqueuses.

Les compositions selon l'invention peuvent par exemple être utilisées comme produits de traitement, de soin, de protection et/ou de nettoyage de la peau sous forme de crèmes ou de laits, ou comme produits de maquillage (peau et lèvres) par incorporation de charges et/ou de matières colorantes (pigments et/ou colorants). Elles sont particulièrement appropriées pour le traitement des rides et/ou ridules de la peau et pour le traitement et/ou la protection de la peau sèche.

Aussi, l'invention a encore pour objet l'utilisation cosmétique de la composition telle que définie ci-dessus pour le traitement, la protection, le soin et/ou le nettoyage de la peau, des muqueuses et/ou les cheveux, et/ou pour le maquillage de la peau et/ou des muqueuses.

Elle a aussi pour objet l'utilisation cosmétique de la composition telle que définie ci-dessus pour le traitement des rides et/ou des ridules de la peau.

L'invention a encore pour objet l'utilisation de la composition telle que définie ci-dessus pour la fabrication d'une composition destinée au traitement et/ou à la protection de la peau sèche.

En outre, de façon connue, les compositions de l'invention peuvent contenir des adjuvants habituels dans le domaine cosmétique ou dermatologique, tels que des actifs hydrophiles ou lipophiles, des conservateurs, des antioxydants, des parfums, des solvants, des filtres solaires, des matières colorantes, des agents basiques ou acides et encore des vésicules lipidiques. Ces adjuvants sont utilisés dans les proportions habituelles dans le domaine cosmétique ou dermatologique, et par exemple de 0,01 à 30 % du poids total de la composition, et ils sont, selon leur nature, introduits dans la phase aqueuse ou dans la phase huileuse de la composition, ou encore dans des vésicules. Ces adjuvants ainsi que leurs concentrations doivent être tels qu'ils ne modifient pas la propriété recherchée pour la composition.

Comme actifs utilisables dans la composition de l'invention, on peut citer par exemple les hydratants tels que les polyols et notamment la glycérine, l'éthylène glycol, l'isoprène-glycol, le propane-1,2 diol, la diglycérine, le sorbitol, les polyéthylène glycols et leurs mélanges.

La composition selon l'invention peut être préparée de manière avantageuse en utilisant, pour au moins une étape du procédé, un appareil de malaxage tel qu'un broyeur à cylindres comportant deux cylindres tournant en sens inverse entre lesquels passe la pâte ou un mélangeur-extrudeur à vis. On utilise de préférence un mélangeur-extrudeur à vis.

Un autre objet de l'invention est donc un procédé de préparation d'une composition selon l'invention, caractérisé en ce que l'on effectue au moins une étape du procédé à l'aide d'un mélangeur-extrudeur à vis.

Selon un premier mode de réalisation de l'invention, le procédé de préparation comprend les étapes suivantes :
- (1) préparation de la phase huileuse sous forme d'une pâte souple obtenue en faisant un prémélange des cires et huiles, en chauffant ce prémélange à une température à laquelle il fond, puis en introduisant dans un mélangeur-extrudeur à vis soumis à un gradient de température allant de 80°C à 20°C, en une ou plusieurs fois, le prémélange fondu et les autres constituants (notamment les charges) de la phase huileuse, et en malaxant le mélange obtenu tout en le refroidissant jusqu'à température ambiante tout en l'amenant à la sortie du mélangeur-extrudeur ;
- (2) incorporation de l'émulsionnant siliconé dans la pâte souple obtenue en (1), et
- (3) incorporation, sous agitation, de la phase aqueuse dans le mélange obtenu en (2).

Dans ce mode de réalisation, les étapes (2) et (3) sont réalisées dans un appareil de mélange habituellement utilisé par l'homme du métier, tel qu'un rotor stator.

Par ailleurs, dans le procédé décrit ci-dessus, les quantités utilisées sont telles que l'émulsion obtenue comporte au moins 5 % en poids de cire et au moins 25 % en poids de phase aqueuse, par rapport au poids total de la composition.

Comme indiqué plus haut, le mélange des phases huileuse et aqueuse se faisant à froid, l'incorporation de composés thermosensibles ne pose pas de problème.

Selon un mode particulier de réalisation de l'invention, les étapes (2) et (3) ci-dessus sont réalisées également dans le mélangeur-extrudeur à vis utilisé pour l'étape (1). L'émulsionnant siliconé et la phase aqueuse sont alors introduits dans une partie (ou élément) du mélangeur-extrudeur où la température est proche de la température ambiante.

L'utilisation d'un mélangeur-extrudeur permet d'obtenir de façon reproductible une pâte de phase huileuse de qualité très constante. De plus, il est possible, en adaptant la filière de sortie du mélangeur-extrudeur, de conditionner la composition en ligne à la sortie dudit mélangeur-extrudeur.

Les différents étapes du procédé peuvent être réalisées dans un ou plusieurs extrudeurs disposés à la suite les uns des autres, et de préférence dans un extrudeur bivis unique.

Les conditions dans lesquelles l'extrusion peut être effectuée sont décrites dans le document FR-A-2,715,306 dont le contenu est incorporé à la présente demande par référence.

L'invention est illustrée plus en détail dans l'exemple suivant. Les quantités indiquées sont des pourcentages en poids.

### Exemple : crème de soin

*Phase huileuse*
- Dry-Flo (charge) 7 %
- Cire microcristalline 19 %
- Huile minérale qsp 100 %

*Emulsion E/H*
- Phase huileuse 40 %
- Dimethicone copolyol / cyclomethicone (Q2-3225C) 10 %
- Eau qsp 100 %

### Mode opératoire 1 :

- On chauffe le mélange de cire et d'huile à environ 100°C,
- on introduit le mélange fondu dans un mélangeur-extrudeur en même temps que la charge, on obtient à la sortie du mélangeur-extrudeur la phase huileuse sous forme d'une pâte souple,
- Dans un rotor stator, on incorpore dans la pâte souple l'émulsionnant siliconé,
- on ajoute au mélange peu à peu l'eau tout en agitant.

### Mode opératoire 2:

- On chauffe le mélange de cire et d'huile à environ 100°C,
- on introduit la charge dans l'élément de tête d'un mélangeur-extrudeur comportant au moins six éléments,
- on introduit la phase huileuse dans le second élément dudit mélangeur-extrudeur à vis, et
- on introduit la phase aqueuse et l'émulsionnant siliconé par deux entrées différentes, dans le quatrième élément dudit mélangeur-extrudeur à vis.

Les éléments du mélangeur-extrudeur à vis utilisé sont, en allant du premier au sixième élément, portés respectivement aux températures suivantes : 20°C, 80°C, 60°C, 20°C, 20°C et 20°C.

On obtient une crème qui présente une texture très légère et qui possède de bonnes qualités d'hydratation et est apte à lisser le relief de la peau.

## Revendications

1. Composition sous forme d'une crème constituée d'une émulsion eau-dans-huile comportant une phase aqueuse dispersée dans une phase huileuse, **caractérisée en ce qu'**elle contient au moins 25 % en poids de phase aqueuse par rapport au poids total de la composition, au moins un émulsionnant siliconé et au moins 5 % en poids d'une ou plusieurs cires par rapport au poids total de la composition, **en ce que** la phase huileuse se présente sous forme d'une pâte souple à la température ambiante, susceptible d'être obtenue en malaxant le mélange obtenu à partir du prémélange fondu des cires et huiles et des autres constituants de la phase huileuse tout en le refroidissant jusqu'à température ambiante et **en ce que** le mélange des phases huileuse et aqueuse se fait à froid.

2. Composition selon la revendication 1, **caractérisée en ce que** l'émulsionnant siliconé est choisi dans le groupe comprenant les polydiméthylsiloxanes oxyéthylénés et/ou oxypropylénés, les alkyl-C₁₀-C₂₂-polydiméthylsiloxanes oxyéthylénés et/ou oxypropylénés, les polydimethylsiloxanes oxyéthylénés et/ou oxypropylénés à groupements glucosides, et leurs mélanges.

3. Composition selon la revendication 1 ou 2, **caractérisée en ce que** la quantité d'émulsionnant siliconé va de 0,1 à 10 % en poids de matière active par rapport au poids total de la composition.

4. Composition selon l'une quelconque des revendications précédentes, **caractérisée en ce que** la cire est choisie dans le groupe comprenant les cires minérales, les cires animales, les cires végétales, les huiles hydrogénées concrètes à 25°C, les esters gras et les glycérides concrets à 25°C, les cires synthétiques, les cires de silicone et leurs mélanges.

5. Composition selon l'une quelconque des revendications précédentes, **caractérisée en ce qu'**elle contient au moins une cire choisie parmi les cires ayant une température de fusion commençante supérieure ou égale à 50°C.

6. Composition selon l'une quelconque des revendications précédentes, **caractérisée en ce que** la cire est choisie parmi la cire de Camauba, les cires de polyéthylène ayant une température de fusion commençante supérieure à 65°C, les cires microcristallines ayant une température de fusion commençante supérieure à 65°C, et leurs mélanges.

7. Composition selon l'une quelconque des revendications précédentes, **caractérisée en ce que** la quantité de cire(s) va de 5 à 30 % en poids par rapport au poids total de la composition.

8. Composition selon l'une quelconque des revendications précédentes, **caractérisée en ce que** la quantité de phase huileuse va de 20 à 75 % en poids par rapport au poids total de la composition.

9. Composition selon l'une quelconque des revendications précédentes, **caractérisée en ce que** la phase huileuse comprend, en outre, un ou plusieurs corps gras choisis parmi les huiles d'origine animale, les huiles d'origine végétale, les huiles minérales, les huiles synthétiques, les huiles fluorées, les huiles de silicone et notamment les huiles de silicone volatiles, les gommes de silicone, les résines de silicone, les alcools gras, les acides gras et les élastomères de silicone.

10. Composition selon l'une quelconque des revendications précédentes, **caractérisée en ce qu'**elle comprend au moins une charge.

11. Composition selon la revendication précédente, **caractérisée en ce que** la quantité de charge(s) va de 1 à 12 % en poids par rapport au poids total de la composition.

12. Composition selon l'une quelconque des revendications précédentes, **caractérisée en ce que** la phase aqueuse représente de 40 à 70 % en poids par rapport au poids total de la composition.

13. Composition selon l'une quelconque des revendications précédentes, **caractérisée en ce qu'**elle constitue une composition cosmétique et/ou dermatologique.

14. Utilisation cosmétique de la composition selon l'une quelconque des revendications précédentes pour le traitement, la protection, le soin et/ou le nettoyage de la peau, des muqueuses et/ou les cheveux, et/ou pour le maquillage de la peau et/ou des muqueuses.

15. Utilisation cosmétique de la composition selon l'une quelconque des revendications 1 à 13 pour le traitement des rides et/ou des ridules de la peau.

16. Utilisation de la composition selon l'une quelconque des revendications 1 à 13 pour la fabrication d'une composition destinée au traitement et/ou à la protection de la peau sèche.

17. Procédé de préparation d'une composition selon l'une quelconque des revendications 1 à 13, **caractérisé en ce que** l'on effectue au moins une étape du procédé à l'aide d'un mélangeur-extrudeur à vis.

18. Procédé selon la revendication 17, **caractérisé en ce qu'**il comprend les étapes suivantes :
- (1) préparation de la phase huileuse sous forme d'une pâte souple obtenue en faisant un prémélange des cires et huiles, en chauffant ce prémélange à une température à laquelle il fond, puis en introduisant dans un mélangeur-extrudeur à vis soumis à un gradient de température allant de 80°C à 20°C, en une ou plusieurs fois, le prémélange fondu et les autres constituants de la phase huileuse, et en malaxant le mélange obtenu tout en le refroidissant jusqu'à température ambiante tout en l'amenant à la sortie du mélangeur-extrudeur ;
- (2) incorporation de l'émulsionnant siliconé dans la pâte souple obtenue en (1), et
- (3) incorporation, sous agitation, de la phase aqueuse dans le mélange obtenu en (2).

19. Procédé selon la revendication 17 ou 18, **caractérisé en ce que** les étapes (2) et (3) sont réalisées dans le mélangeur-extrudeur à vis de l'étape (1).

20. Procédé selon l'une quelconque des revendications 17 à 19, **caractérisé en ce que** la phase huileuse se présente sous forme d'une pâte souple ayant une viscosité dynamique à 25°C comprise entre 3 et 35 Pa.s, mesurée avec un viscosimètre rotatif CONTRAVES TV équipé d'un mobile "MS-r4" à la fréquence de 60 Hz.

## Patentansprüche

1. Zusammensetzung, die in Form eine Creme vorliegt und eine Wasser-in-Öl-Emulsion darstellt, die eine in einer Ölphase dispergierte wäßrige Phase enthält, **dadurch gekennzeichnet, daß** sie mindestens 25 Gew,-% wäßrige Phase, bezogen auf das Gesamtgewicht der Zusammensetzung, mindestens einen Siliconemulgator und mindestens 5 Gew.-% eines oder mehrerer Wachse, bezogen auf das Gesamtgewicht der Zusammensetzung, enthält, dadurch, daß die Ölphase in Form einer bei Raumtemperatur weichen Paste vorliegt und hergestellt werden kann, indem das Gemisch, das aus dem geschmolzenen Vorgemisch der Wachse und Öle und weiteren Bestandteile der Ölphase erhalten wird, geknetet und bis auf Raumtemperatur abgekühlt wird, und dadurch, daß das Mischen von Ölphase und wäßriger Phase in der Kälte erfolgt.

2. Zusammensetzung nach Anspruch 1, **dadurch gekennzeichnet, daß** der Siliconemulgator unter den ethoxylierten und/oder propoxylierten Polydimethylsiloxanen, ethoxylierten und/oder propoxylierten C₁₀₋₂₂-Alkylpolydimethylsiloxanen, ethoxylierten und/oder propoxylierten Polydimethylsiloxanen mit Glucosidgruppen und deren Gemischen ausgewählt ist.

3. Zusammensetzung nach Anspruch 1 oder 2, **dadurch gekennzeichnet, daß** die Menge des Siliconemulgators im Bereich von 0,1 bis 10 Gew.-% Wirkstoff, bezogen auf das Gesamtgewicht der Zusammensetzung, liegt.

4. Zusammensetzung nach einem der vorhergehenden Ansprüche, **dadurch gekennzeichnet, daß** das Wachs unter den mineralischen Wachsen, tierischen Wachsen, pflanzlichen Wachsen, bei 25 °C festen, hydrierten Ölen, Fettsäureestem und Glyceriden, die bei 25 °C fest sind, synthetischen Wachsen, Siliconwachsen und deren Gemischen ausgewählt ist.

5. Zusammensetzung nach einem der vorhergehenden Ansprüche, **dadurch gekennzeichnet, daß** sie mindestens ein Wachs enthält, das unter den Wachsen ausgewählt ist, die eine beginnende Schmelztemperatur von mindestens 50 °C aufweisen.

6. Zusammensetzung nach einem der vorhergehenden Ansprüche, **dadurch gekennzeichnet, daß** das Wachs unter Carnaubawachs, den Polyethylenwachsen, die eine beginnende Schmelztemperatur über 65°C aufweisen, den mikrokristallinen Wachsen, die eine beginnende Schmelztemperatur über 65 °C aufweisen, und deren Gemischen ausgewählt ist.

7. Zusammensetzung nach einem der vorhergehenden Ansprüche, **dadurch gekennzeichnet, daß** die Menge des Wachses oder der Wachse im Bereich von 5 bis 30 Gew.-%, bezogen auf das Gesamtgewicht der Zusammensetzung, liegt.

8. Zusammensetzung nach einem der vorhergehenden Ansprüche, **dadurch gekennzeichnet, daß** die Menge der Ölphase im Bereich von 20 bis 75 Gew.-%, bezogen auf das Gesamtgewicht der Zusammensetzung, liegt.

9. Zusammensetzung nach einem der vorhergehenden Ansprüche, **dadurch gekennzeichnet, daß** die Ölphase eine oder mehrere Fettsubstanzen enthält, die unter den Ölen tierischer Herkunft, den Ölen pflanzlicher Herkunft, den Mineralölen, den synthetischen Ölen, den fluorierten Ölen, den Siliconölen und insbesondere den flüchtigen Siliconölen, den Silicongummis, den Siliconharzen, den Fettalkoholen, den Fettsäuren und den Siliconelastomeren ausgewählt sind.

10. Zusammensetzung nach einem der vorhergehenden Ansprüche, **dadurch gekennzeichnet, daß** sie mindestens einen Füllstoff enthält.

11. Zusammensetzung nach dem vorhergehenden Anspruch, **dadurch gekennzeichnet, daß** die Menge des Füllstoffes oder der Füllstoffe im Bereich von 1 bis 12 Gew.-%, bezogen auf das Gesamtgewicht der Zusammensetzung, liegt.

12. Zusammensetzung nach einem der vorhergehenden Ansprüche, **dadurch gekennzeichnet, daß** die wäßrige Phase 40 bis 70 Gew.-%, bezogen auf das Gesamtgewicht der Zusammensetzung, ausmacht.

13. Zusammensetzung nach einem der vorhergehenden Ansprüche, **dadurch gekennzeichnet, daß** sie eine kosmetische und/oder dermatologische Zusammensetzung ist.

14. Kosmetische Verwendung der Zusammensetzung nach einem der vorhergehenden Ansprüche zur Behandlung, zum Schutz, zur Pflege und/oder zur Reinigung der Haut, der Schleimhäute und/oder der Haare und/oder zum Schminken der Haut und/oder der Schleimhäute.

15. Kosmetische Verwendung der Zusammensetzung nach einem der Ansprüche 1 bis 13 zur Behandlung von Falten und/oder Fältchen der Haut.

16. Verwendung der Zusammensetzung nach einem der Ansprüche 1 bis 13 zur Herstellung einer Zusammensetzung, die zur Behandlung und/oder zum Schutz von trockener Haut vorgesehen ist.

17. Verfahren zur Herstellung einer Zusammensetzung nach einem der Ansprüche 1 bis 13, **dadurch gekennzeichnet, daß** mindestens ein Verfahrensschritt mit einem Schneckenextrudermischer durchgeführt wird.

18. Verfahren nach Anspruch 17, **dadurch gekennzeichnet, daß** es die folgenden Schritte umfaßt:
(1) Herstellung der Ölphase in Form einer weichen Paste, die erhalten wird, indem ein Vorgemisch von Wachsen und Ölen hergestellt wird, das Vorgemisch auf eine Temperatur erwärmt wird, bei der es schmilzt, das geschmolzene Vorgemisch und die weiteren Bestandteile der Ölphase dann auf einmal oder portionsweise in einen Schneckenextrudermischer mit einem Temperaturgradienten von 80 °C bis 20 °C gegeben werden, und das erhaltene Gemisch unter Abkühlung bis Raumtemperatur geknetet wird, wobei es zum Auslaß des Extrudermischers gebracht wird;
(2) Einarbeiten des Siliconemulgators in die in Schritt (1) hergestellte weiche Paste, und
(3) Einarbeiten der wäßrigen Phase in das in Schritt (2) erhaltene Gemisch unter Rühren.

19. Verfahren nach Anspruch 17 oder 18, **dadurch gekennzeichnet, daß** die Schritte (2) und (3) in dem Schneckenextrudermischer des Schritts (1) durchgeführt werden.

20. Verfahren nach einem der Ansprüche 17 bis 19, **dadurch gekennzeichnet, daß** die Ölphase in Form einer weichen Paste vorliegt, die bei 25 °C eine dynamische Viskosität von 3 bis 35 Pa·s aufweist, wobei die Viskosität mit einem Rotationsviskosimeter CONTRAVES TV, das mit einem mobilen Teil "MS-r4" ausgestattet ist, bei einer Frequenz von 60 Hz ermittelt wird.

## Claims

1. Composition in the form of a cream composed of a water-in-oil emulsion comprising an aqueous phase dispersed in an oily phase, **characterized in that** it comprises at least 25% by weight of aqueous phase with respect to the total weight of the composition, at least one silicone emulsifier and at least 5% by weight of one or more waxes with respect to the total weight of the composition, **in that** the oily phase exists in the form of a soft paste at ambient temperature capable of being obtained by kneading the mixture obtained from the molten premix of the waxes and oils and of the other constituents of the oily phase while cooling it to ambient temperature and **in that** the mixing of the oily and aqueous phases takes place under cold conditions.

2. Composition according to Claim 1, **characterized in that** the silicone emulsifier is chosen from the group comprising oxyethylenated and/or oxypropylenated polydimethylsiloxanes, oxyethylenated and/or oxypropylenated (C₁₀-C₂₂) alkylpoly-dimethylsiloxanes, oxyethylenated and/or oxypropylenated polydimethylsiloxanes with glucoside groups, and their mixtures.

3. Composition according to Claim 1 or 2, **characterized in that** the amount of silicone emulsifier ranges from 0.1 to 10% by weight of active material with respect to the total weight of the composition.

4. Composition according to any one of the preceding claims, **characterized in that** the wax is chosen from the group comprising mineral waxes, animal waxes, vegetable waxes, hydrogenated oils which are solid at 25°C, fatty esters and glycerides which are solid at 25°C, synthetic waxes, silicone waxes, and their mixtures.

5. Composition according to any one of the preceding claims, **characterized in that** it comprises at least one wax chosen from waxes having a starting melting temperature of greater than or equal to 50°C.

6. Composition according to any one of the preceding claims, **characterised in that** the wax is chosen from carnauba wax, polyethylene waxes having a starting melting temperature of greater than 65°C, microcrystalline waxes having a starting melting temperature of greater than 65°C, and their mixtures.

7. Composition according to any one of the preceding claims, **characterized in that** the amount of wax(es) ranges from 5 to 30% by weight with respect to the total weight of the composition.

8. Composition according to any one of the preceding claims, **characterized in that** the amount of oily phase ranges from 20 to 75% by weight with respect to the total weight of the composition.

9. Composition according to any one of the preceding claims, **characterized in that** the oily phase additionally comprises one or more fatty substances chosen from oils of animal origin, oils of vegetable origin, mineral oils, synthetic oils, fluorinated oils, silicone oils, in particular volatile silicone oils, silicone gums, silicone resins, fatty alcohols, fatty acids and silicone elastomers.

10. Composition according to any one of the preceding claims, **characterized in that** it comprises at least one filler.

11. Composition according to the preceding claim, **characterized in that** the amount of filler(s) ranges from 1 to 12% by weight with respect to the total weight of the composition.

12. Composition according to any one of the preceding claims, **characterized in that** the aqueous phase represents from 40 to 70% by weight with respect to the total weight of the composition.

13. Composition according to any one of the preceding claims, **characterized in that** it constitutes a cosmetic and/or dermatological composition.

14. Cosmetic use of the composition according to any one of the preceding claims in treating, protecting, caring for and/or cleansing the skin, mucous membranes and/or hair and/or in making up the skin and/or mucous membranes.

15. Cosmetic use of the composition according to any one of Claims 1 to 13 in treating wrinkles and/or fine lines of the skin.

16. Use of the composition according to any one of Claims 1 to 13 in the manufacture of a composition intended for treating and/or protecting dry skin.

17. Process for the preparation of a composition according to any one of Claims 1 to 13, **characterized in that** at least one stage of the process is carried out using a screw mixer-extruder.

18. Process according to Claim 17, **characterized in that** it comprises the following stages:
- (1) preparation of the oily phase in the form of a soft paste obtained by forming a premix of the waxes and oils, by heating this premix to a temperature at which it melts, by then introducing the molten premix and the other constituents of the oily phase, all at once or in several portions, into a screw mixer-extruder subject to a temperature gradient ranging from 80°C to 20°C, and by kneading the mixture obtained while cooling it to ambient temperature while conveying it to the outlet of the mixer-extruder;
- (2) incorporation of the silicone emulsifier in the soft paste obtained in (1), and
- (3) incorporation, with stirring, of the aqueous phase in the mixture obtained in (2).

19. Process according to Claim 17 or 18, **characterized in that** stages (2) and (3) are carried out in the screw mixer-extruder of stage (1).

20. Process according to any one of Claims 17 to 19, **characterized in that** the oily phase exists in the form of a soft paste having a dynamic viscosity at 25°C of between 3 and 35 Pa·s, measured with a Contraves TV rotary viscometer equipped with an "MS-r4" rotor at a frequency of 60 Hz.
